# EUROPEAN PATENT SPECIFICATION

(11) **EP 0 964 722 B1**
(45) Date of publication and mention of the grant of the patent: **01.09.2004**
(21) Application number: 97950357.0
(22) Date of filing: 23.12.1997
(51) Int. Cl.: A61N 1/30, A61N 1/04

(54) **ELECTRODE ASSEMBLY FOR A DEVICE FOR THE DELIVERY OF A SUBSTANCE TO A SUBJECT**
ELEKTRODENEINHEIT FÜR EINE VORRICHTUNG ZUR VERABREICHUNG VON MEDIKAMENTEN
ENSEMBLE D'ELECTRODES POUR UN DISPOSITIF D'ADMINISTRATION D'UNE SUBSTANCE A UN SUJET

(30) Priority: 26.12.1996 US 34363 P; 28.01.1997 US 36514 P; 30.07.1997 IE 970557
(43) Date of publication of application: 22.12.1999
(73) Proprietor: Elan International Services Limited, Hamilton (BM)
(72) Inventor: NITZAN, Zvi, 49600 Petah-Tikva (IL); GROSS, Joseph, Dublin 3 (IE)
(74) Representative: Ryan, Anne Mary
(86) International application number: PCT/IE1997/000089
(87) International publication number: WO 1998/029157

(56) References cited:
- EP-A- 0 060 452
- EP-A- 0 337 642
- WO-A-92/04937
- WO-A-92/17239
- WO-A-94/17853
- WO-A-96/10440
- WO-A-96/30077
- DE-A- 4 028 125
- GB-A- 2 239 803

## Description

### Technical Field

This invention relates to an improved electrode assembly for use in devices for the delivery of a substance to a subject, and, in particular, to devices for the delivery of a substance *via* the skin of a subject.

### Background Art

Typical modes of delivery *via* the skin of a subject include transdermal, iontophoretic, intradermal, subcutaneous, intravenous and intramuscular delivery.

In many cases where a substance is to be delivered at a controlled rate, the device requires electronic controlling circuitry and delivery or driving means for effecting delivery, under the influence of the controlling circuitry, of the substance from a reservoir to the subject.

The prior art includes a number of devices in which the electronic controller is reusable and the reservoir and delivery means are disposable. This enables the more expensive electronic components to be re-used on a number of occasions, with the disposable part being replaced as the supply of substance becomes exhausted.

In general, the two parts of such devices are provided in separate housing elements which are adapted to connect together by means of a snap-fit connection, for example. A problem associated with such devices is that they are unsuitable for applying a substance such as a medicament or a cosmetic substance to a large area of skin since it is difficult to design a housing which conforms to a large area of skin for a range of subjects, particularly if a degree of mobility is required while the device is in place.

A further disadvantage associated with such devices is the cost associated with designing and manufacturing the components of a housing which must fit together reliably.

The present invention aims to provide an electrode assembly for use in a device which overcomes these problems and which is significantly simpler in design and thus less expensive and easier to manufacture, as well as being more user friendly, than known devices.

### Disclosure of Invention

Thus, the invention provides an improved electrode assembly for use in a device for the delivery of a substance to a subject, comprising:
a) a flexible substrate having a first surface and a second opposed surface;
b) a pair of spaced apart electrodes applied to the first surface of the substrate;
c) a tab extending from each electrode comprised of electrically conductive material; and
d) a power source located within a housing having a pair of spaced apart electrical contacts located on the bottom surface of the housing;
whereby the tabs are folded over onto the opposed surface of the substrate and the power source is fixed to the opposed surface so that each electrical contact is in electrical communication with one of the tabs.

Preferably, the substrate comprises a non-woven tissue paper.

Preferably, the electrodes are comprised of electrically conductive ink.

Further, preferably, the electrodes are printed onto the substrate.

Preferably, the power source is fixed to the opposed surface of the substrate by means of an electrically conductive adhesive.

Preferably, the assembly further comprises means for retaining the substrate to the skin of a mammal.

Preferably, the retaining means is an adhesive.

Further, preferably, the power source further comprises an electrical circuit capable of generating an alternating voltage.

Preferably, the voltage alternates between the electrodes.

Preferably, said voltage alternates with a period of from about 20 seconds to about 10 minutes.

More preferably, said voltage alternates with a period of from about 1 minute to about 5 minutes.

This timescale has been found to provide good delivery rates without leading to an overloading of substance in the area of skin immediately below the electrode. It will be appreciated that for individual substances and individual delivery rates or regimes, alternative periods may be suitable, or direct current delivery mode may be preferable.

Preferably, said alternating voltage defines a substantially square waveform having a sawtooth component during the transition between polarities.

The sawtooth component eliminates abrupt transitions between polarities and this has been found to make the device more comfortable for users as it eliminates the "tingling" sensation sometimes associated with AC iontophoretic delivery. Typically, the time taken for the transition between polarities is of the order of 0.1-10 seconds, preferably 0.5-5 seconds.

### Brief Description of Drawings

The invention will be further illustrated by the following description of an embodiments thereof, given by way of example only with reference to the accompanying drawings, in which:
Fig. 1 is an exploded perspective view of a first preferred embodiment of a device in which an electrode assembly according to the invention can be used;
Fig. 2 is a perspective view of the underside of the first part of the embodiment of Fig. 1;
Fig. 3 is a further exploded perspective view of the embodiment of Fig. 1, with some components assembled together;
Fig. 4 is a perspective view of the embodiment of Fig. 1, when fully assembled;
Fig. 5 is a perspective view of a second preferred embodiment, when fully assembled, of a device in which an electrode assembly according to the invention can be used.
Fig. 6 is a partially exploded perspective view of the embodiment of Fig. 5;
Fig. 7 is a perspective view of the underside of the first part of the embodiment of Fig. 5;
Fig. 8 is a partially exploded perspective view of a third preferred embodiment of a device in which an electrode assembly according to the invention can be used;
Fig. 9 is a perspective view of the underside of the first part of the embodiment of Fig. 8;
Fig. 10 is a perspective view of the embodiment of Fig. 8, when fully assembled;
Fig. 11 is a perspective view of the embodiment of Fig. 8, in use;
Fig. 12 is a perspective view of a fourth preferred embodiment, when fully assembled, of a device in which an electrode assembly according to the invention can be used;
Fig. 13 is an exploded perspective view of a fifth preferred embodiment of a device in which an electrode assembly according to the invention can be used;
Fig. 14 is a perspective view of the underside of the first part of the embodiment of Fig. 13;
Fig. 15 is a perspective view of the embodiment of Fig. 13, when fully assembled;
Fig. 16 is a block diagram of a circuit used in the controlling means of a preferred embodiment of a device described herein;
Fig. 17 is a schematic graphical illustration of the voltage waveform produced by the circuit of Fig. 16, shown as voltage plotted against time;
Fig. 18 is a perspective view of a sixth preferred embodiment of a device in which an electrode assembly according to the invention can be used;
Fig. 19 is a perspective view of a seventh preferred embodiment of a device in which an electrode assembly according to the invention can be used, being a variation on the embodiment of Fig. 18;
Fig. 20 is a perspective view of a detail of the embodiment of Fig. 19;
Fig. 21 is a perspective view of the underside of an electrode assembly according to the invention; and
Fig. 22 is a perspective view of the top of the electrode assembly of Fig. 21.

### Modes for Carrying out the Invention

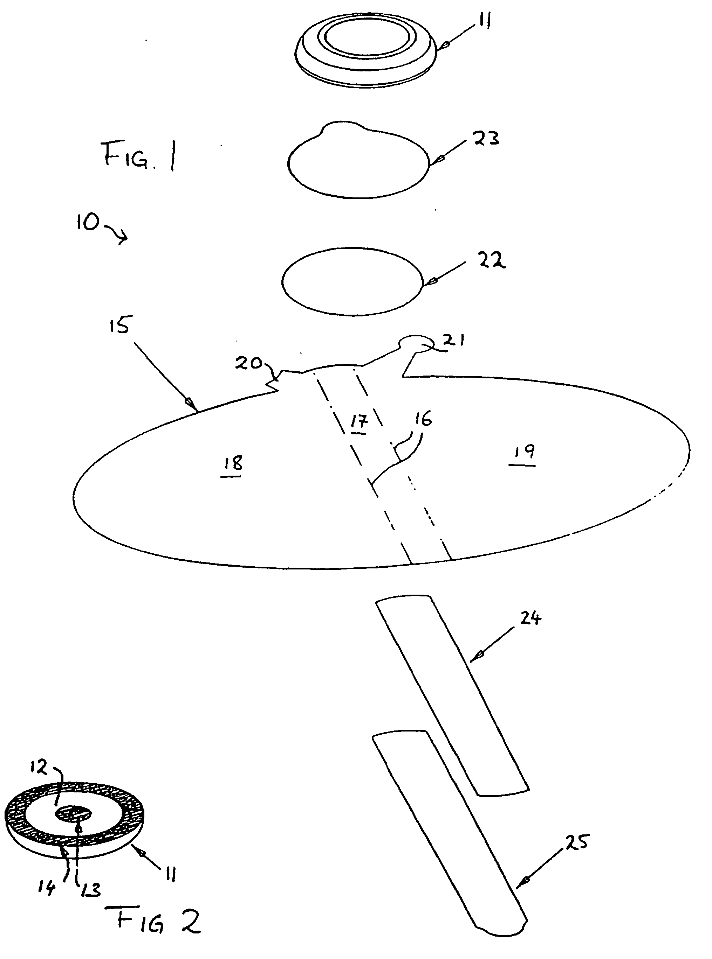

Figs. 1-20, although not illustrating embodiments of an improved electrode assembly according to the invention, are included for reasons of clarity.

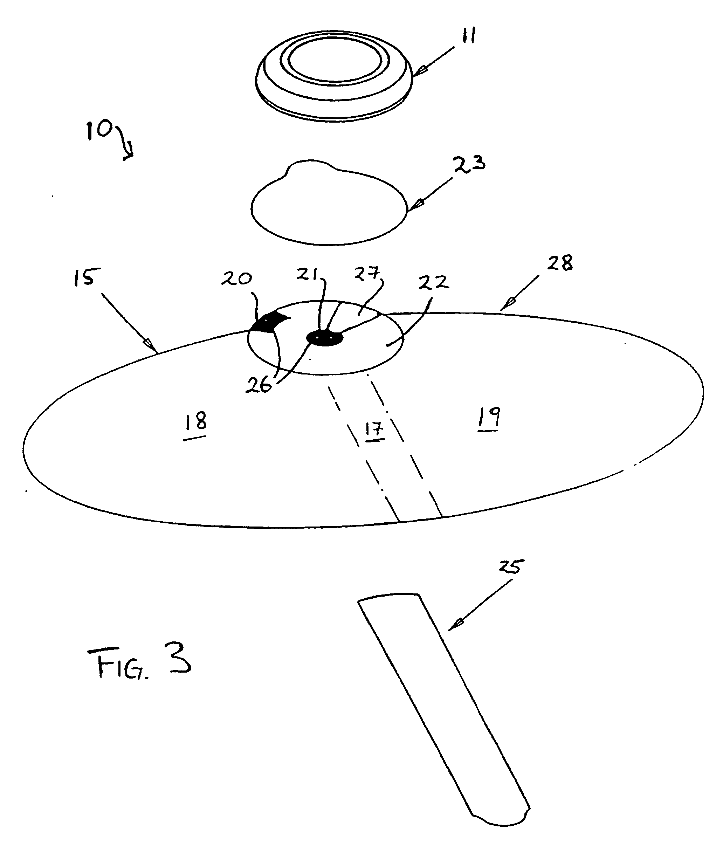

In Fig. 1 there is indicated, generally at 10, an exploded view of a device of the type in which an improved electrode assembly according to the invention would be used. The device 10 comprises a first part 11 which includes a battery and associated circuitry (described below in relation to Figs. 16 and 17) for generating and maintaining a predetermined current profile when connected to delivery means. Fig. 2 shows the underside surface 12 of first part 11, on which a pair of concentric metallic contacts, namely a central contact 13 and an annular contact 14, are disposed.

Referring back to Fig. 1, there is also shown a flexible substrate 15 of insulating material such as hygienic non-woven tissue paper. One preferred material is a 60 grade tissue paper made by EFAR of Tiberius, Israel. The underside (not visible) of sheet 15 is coated with a food grade lacquer and this lacquer in turn is coated with an electrically conductive coating using conductive ink. One preferred conductive ink is made by adding, to a conductive ink (available from Sipca of Switzerland) an amount of carbon powder sufficient to provide the required conductivity. One preferred carbon powder is "Super P" carbon powder manufactured by M.M.M. Carbon of Willebrock, Belgium.

A central strip, denoted by dotted lines 16, on the underside of sheet 15, is not coated with conductive ink 26, thus leaving an insulating barrier 17 between two halves 18,19 of the conductive underside.

The sheet 15 is shaped such that a short tab 20 and a long tab 21 project from the edge of the sheet 15. Short tab 20 projects from half 18, and long tab 21 projects from half 19 of the sheet 15. The conductive coating on the underside of each half 18,19 extends to the underside of each of the tabs 20,21, and the tabs 20,21 are electrically isolated from one another by insulating barrier 17.

As shown in the exploded view of Fig. 1, a circular layer of electrically insulating adhesive 22 is located above sheet 15 adjacent the tabs 20,21. A circular release liner 23 is provided above insulating adhesive 22.

As also shown in Fig. 1, a longitudinal layer of electrically insulating adhesive 24 is located below the insulating barrier 17 of sheet 15 and a longitudinal release liner 25 is provided below insulating adhesive 24.

Fig. 3 shows the device 10 in a more advanced state of assembly, with adhesive layer 22 positioned on sheet 15 and the tabs 20,21 folded onto adhesive layer 22 such that the conductive ink coating 26 is uppermost. Long tab 21 is provided with an insulating coating 27 over part of its surface. Release liner 23 is affixed on top of the adhesive layer 22 and the exposed tab surfaces.

The longitudinal layer of insulating adhesive 24 shown in Fig. 1 (not visible in Fig. 3) is positioned on the underside of sheet 15 covering the barrier 17, and longitudinal release liner 25 is affixed to the longitudinal adhesive layer 24 at the final stage of manufacture.

The device 10 is supplied to users with the release liners 23,25 in place, and the first part 11 separate from the second part 28 (second part 28 being the assembly comprising sheet 15 and adhesive layers 22,24).

In use, the device 10 is adapted to deliver a therapeutic or cosmetic agent to the skin of a subject. The substance to be delivered is suitably provided as a cream, oil, lotion or other such agent and is coated on the underside of sheet 15 so as to substantially cover the underside of sheet 15. Release liner 25 is then peeled away so that the central adhesive strip 24 is free of agent but the two halves on either side are covered by the agent which contains the substance for delivery. Sheet 15 is then applied to the area of skin to be treated, with the agent between the skin and the sheet 15.

Preferably, such cosmetic agents include vitamin A and/or vitamin E, or alpha hydroxy acid; and a medicinal agent may include tetracycline, other antibiotics, anti-acne medicaments or anti-toxins.

To reduce cellulite deposits, a user would apply an agent used to reduce cellulite deposits such as caffeine extract, theophylline extract, ginkgo extract, silisium, magnesium, and/or gola.

Applicant also anticipates that agents used in such devices may include those used to treat skin disorders. Agents applied to the skin to treat such disorders include but are not limited to vitamin A, vitamin E, anti-fungal agents and alpha hydroxy acid. Moreover, it is further anticipated that the devices would also include devices and methods for treating skin disorders of the feet, toes and toe nails. These include but are not limited to athlete's foot medicament, anti-fungal agents and skin moisturizers.

Applicant further anticipates similar application of the devices described herein to treat scalp disorders such as psoriasis, and baldness. Agents used in the treatment of head and scalp disorders include: minoxidil, anti-dandruff agent, and anti-psoriasis medicament. It is also anticipated that the devices be applied to the skin surface of the penis to treat male impotency. Agents used in the treatment of male impotency include penile erection stimulants such as prostaglandin. It is anticipated that the devices have numerous applications to a variety of body parts to treat a number of different ailments.

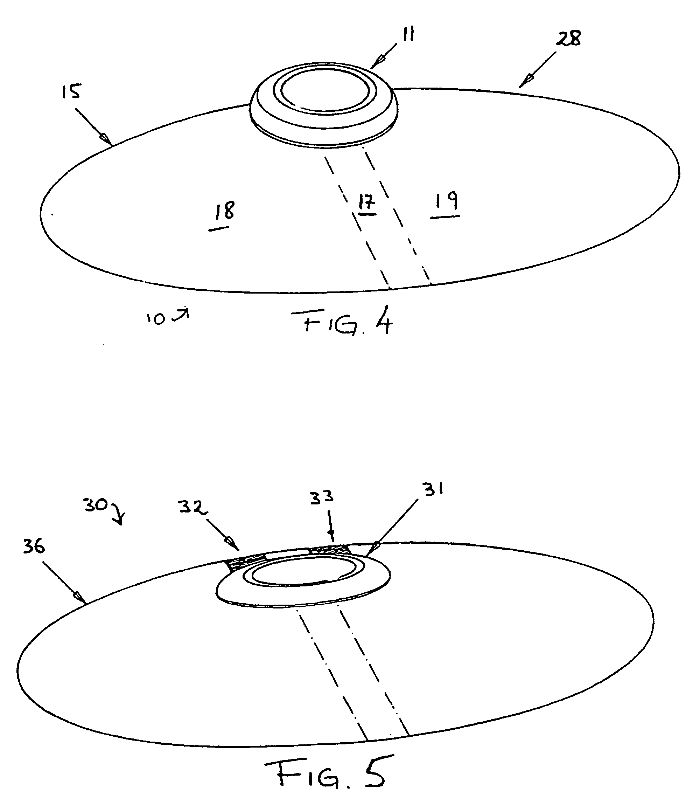

Circular release liner 23 is removed, thereby exposing circular adhesive layer 22 and the conductive surfaces 26 of the short and long tabs 20,21. Circular adhesive layer 22 is designed to hold first part 11 in contact with the conductive surfaces 26 on long tab 21 and short tab 20. Referring additionally to Fig. 2, it can be seen that when first part 11 is applied to the circular adhesive layer 22 (as shown in Fig. 4), central contact 13 (Fig. 2) contacts the coated surface 26 (Fig. 3) of long tab 21, and annular contact 14 (Fig. 2) contacts the coated surface 26 (Fig. 3) of short tab 20.

An iontophoretic electrical circuit may thus be established from the central contact 12 to the annular contact 14 of first part 11 *via* the skin of the subject. The iontophoretic circuit utilises the agent-coated conducting underside of one half of the sheet 15 as a working electrode and the agent-coated conducting underside of the other half of the sheet 15 as a counter electrode. The principles of such iontophoretic delivery are well known to those skilled in the art.

By employing suitable circuitry in first part 11, the direction of flow of current can be arranged to alternate such that each half 18,19 of the underside of sheet 15 repeatedly alternates between acting as the working electrode and the counter electrode. This reduces the likelihood of burns, and enables one to effectively utilise the entire area of the sheet 15 for delivery of the substance (apart of course from the small area of the insulating barrier 17 which separates the two halves 18,19).

The advantages of the device 10 can be appreciated in that it is lightweight, inexpensive, and extremely easy to apply. The method of attaching the electronic controller of first part 11 to the electrode sheet 15 of second part 28 is particularly advantageous, since by simply applying the first part 11 onto the contact adhesive 22, the necessary electrical connections are automatically made and the device is ready for delivery of drug.

Fig. 5 shows a first alternative embodiment, indicated generally at 30, which is essentially similar to device 10 of Figs. 1-4, but differs in certain respects. It can be seen that instead of employing central and annular contacts, the device 30 has a first part 31 in contact with a pair of parallel tabs 32,33 which effect contact with two halves 34,35 of the underside (not shown) of the sheet 36. The halves 34,35 are coated with electrically conductive ink as described above.

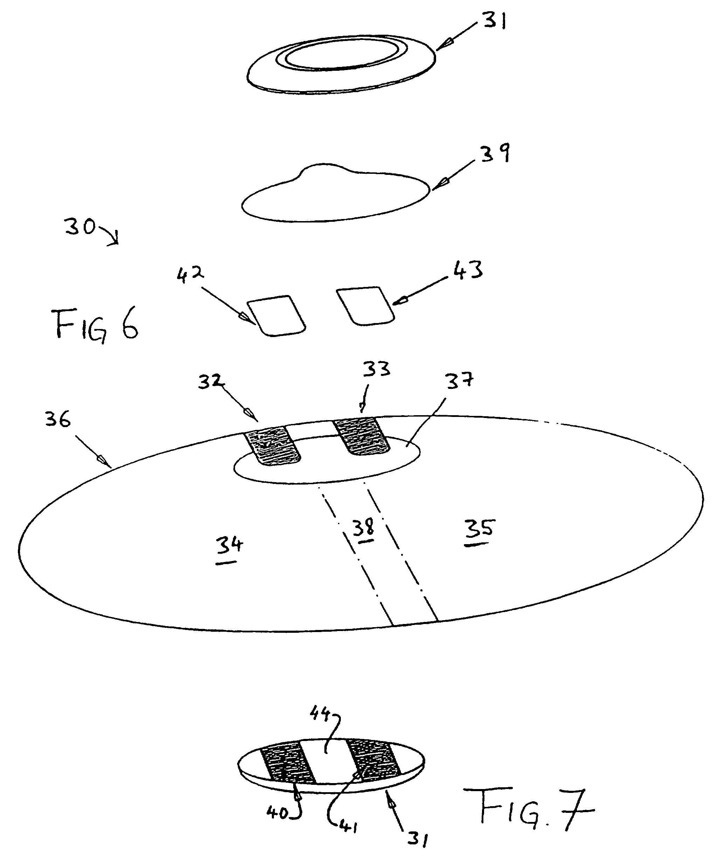

Fig. 6 shows the device 30 in partially exploded view, with a circular insulating adhesive layer 37 already in place on sheet 36, and with tabs 32,33 already folded over onto circular adhesive layer 37. Similarly, a longitudinal insulating adhesive layer (not shown) is already positioned on the underside of insulating barrier 38 along the gap between the coated halves 34,35 of the underside of sheet 36. A release liner (not shown) is provided over the longitudinal adhesive strip (as in the device of Fig. 1-4) and a circular release liner 39 is provided to cover the circular adhesive layer 37.

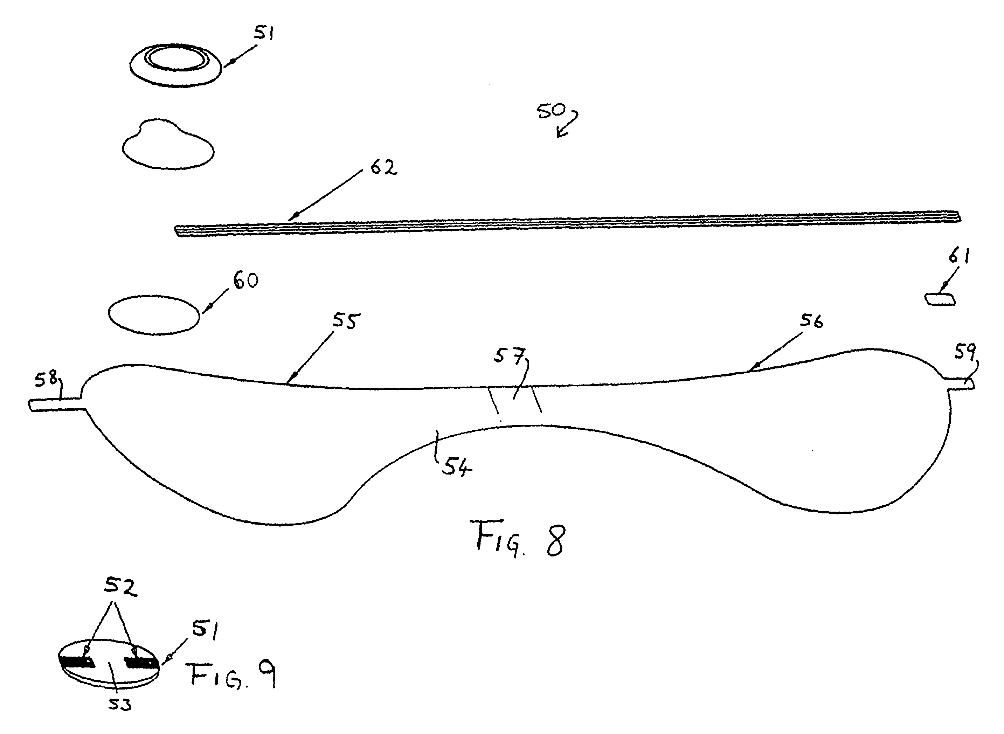

Fig. 7 shows the underside of first part 31 of device 30, from which it can be seen that two parallel electrical contacts 40,41 are dimensioned to contact the parallel conductive tabs 32,33 of sheet 36. Unlike the first part 11 of device 10 (see Fig. 2), first part 31 of device 30 should be oriented correctly to ensure good contact between the contacts 40,41 and the conductive tabs 32,33. However, by choosing a suitable geometry for the tabs and contacts, such difficulties are easily avoided.

A further point of difference from the device 10 of Figs. 1-4 is that a pair of strips of electrically conductive adhesive 42,43 are disposed on top of the conductive tabs 32,33, respectively. A suitable conductive adhesive is sold under item no. 021200-67868 by 3M Corporation.

The strips of conductive adhesive 42,43 ensure a better electrical contact between the contacts 40,41 and the conductive tabs 32,33, than is obtained by relying on mechanical contact alone, and ensure a better adhesion since the entire area of the base 44 (Fig. 7) of first part 31 is adhered to the sheet 36, including the electrical contacts 40,41.

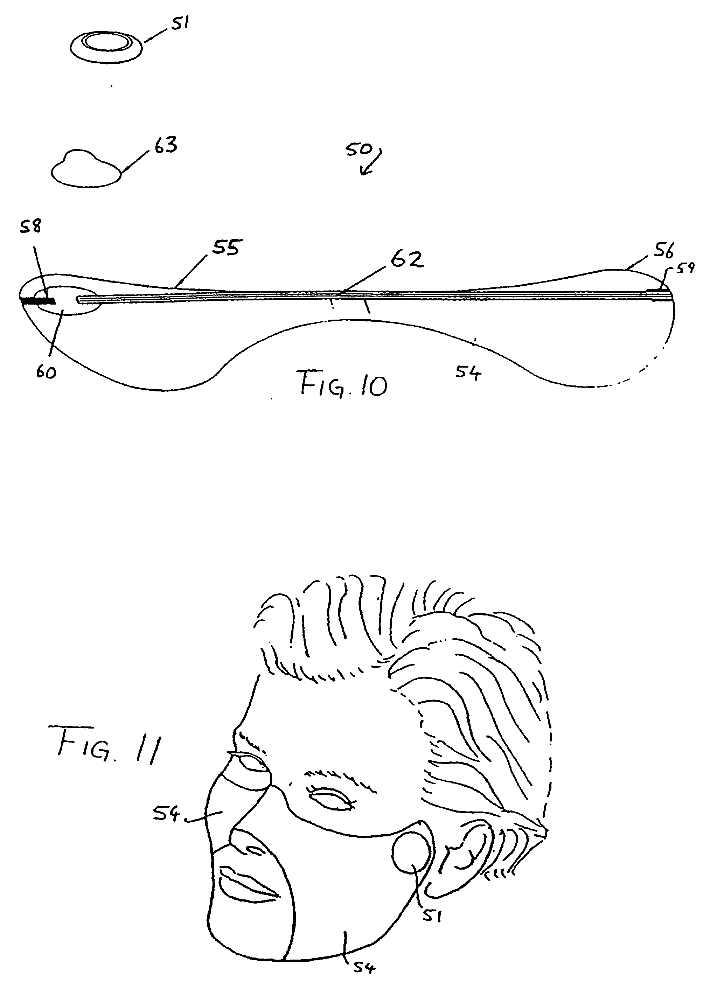

Fig. 8 shows a further embodiment of a device, indicated generally at 50 in exploded view. Device 50 is particularly adapted for cosmetic use, due to the overall shape of the sheet 54 being suitable for application to the nose and cheek area of the face. In this embodiment, the first part 51 has two contacts 52 (see Fig. 9) which are co-linear and separated by a gap 53.

The tissue paper sheet 54 again has two halves 55,56 the undersides of which are coated with conductive ink and separated by an insulating barrier 57 between said halves. From each of the halves 55,56 extends a foldable tab 58,59, respectively.

A circular insulating adhesive layer 60 is used to hold tab 58 in a folded position such that it presents a conducting surface for contact with the first part. A short adhesive strip 61 holds tab 59 in a folded position, and an elongated conductive strip 62, made of metallised polyethylene sheeting having an adhesive underside, is adhered to sheet 54 such that it extends from tab 59 (with which it is in electrical contact) to circular insulating adhesive layer 60, as shown in Fig. 10.

A release liner 63 provided on circular insulating adhesive layer 60 is removed before use, and as described above, the first part 51 is adhered to the circular insulating adhesive layer, whereby one of the contacts 52 on the underside of first part 51 (see Fig. 9) contacts tab 58 (to thereby make an electrical connection with the conductive coating on the underside of half 55 of sheet 54), and the other of the contacts 52 on the underside of first part 51 (see Fig. 9) contacts strip 62 (to thereby make an electrical connection with the conductive coating on the underside of half 56 of sheet 54).

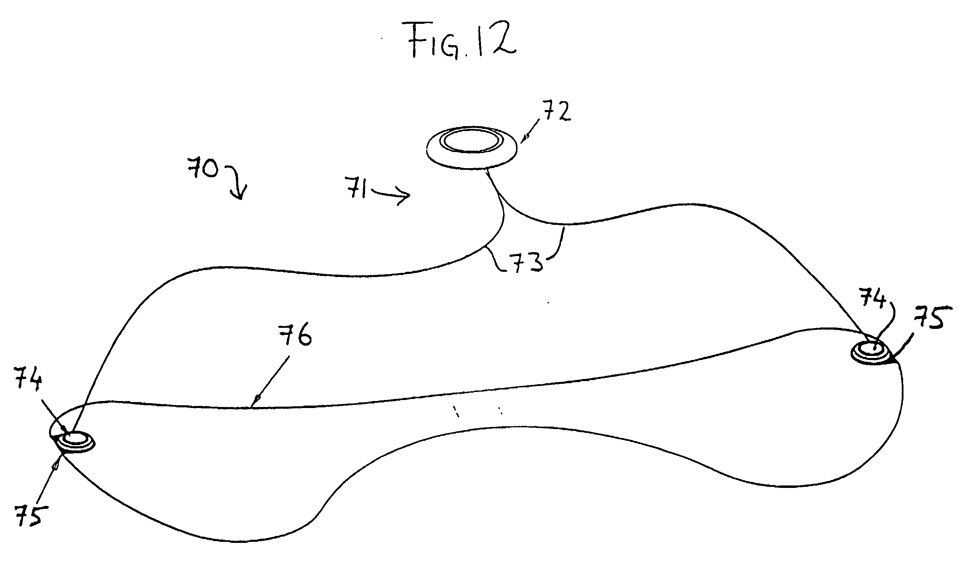

Fig. 11 is a schematic illustration of the device 50 of Fig. 10 in use, with the sheet 54 in place over the cheeks and nose of a user. Although there is an adhesive strip providing some adhesion along the nose (i.e. an adhesive strip on the underside of insulating barrier 57 (Fig. 8), the main adhesive force occurs naturally by as the underside surface of the sheet 54 covered in a cosmetic agent or pharmacologic agent clings to the skin of the face. The first part 51 can be seen beside the ear of the user.

Fig. 12 shows another alternative device, indicated generally at 70. This device differs from that of previously described embodiments in that the first part, indicated generally at 71, has an electronic control module 72 connected by wires 73 to a pair of contacts 74.

Each contact 74 is adhered to a folded tab 75 on tissue sheet 76. The tab is maintained in a folded position by an area of insulating contact adhesive (not shown), and the exposed surface of the tab 75 (i.e. the surface to which the contact 74 is attached) is provided with a layer of electrically conductive adhesive (not shown) to effect both adhesion and electrical contact between the contact 74 and the tab 75. When worn on the face, the appearance is similar to that of the device of Fig. 11, apart from the fact that instead of a single electronic controller positioned on the sheet 76, a pair of smaller contacts 74 extend to either edge of the sheet 76, the visual effect being somewhat similar to a person wearing a pair of headphones.

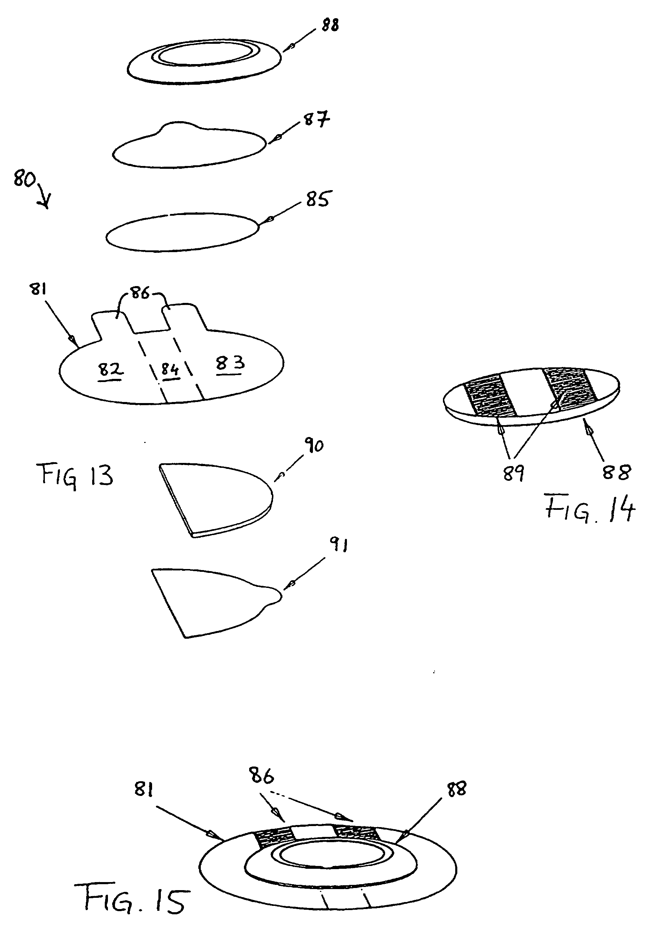

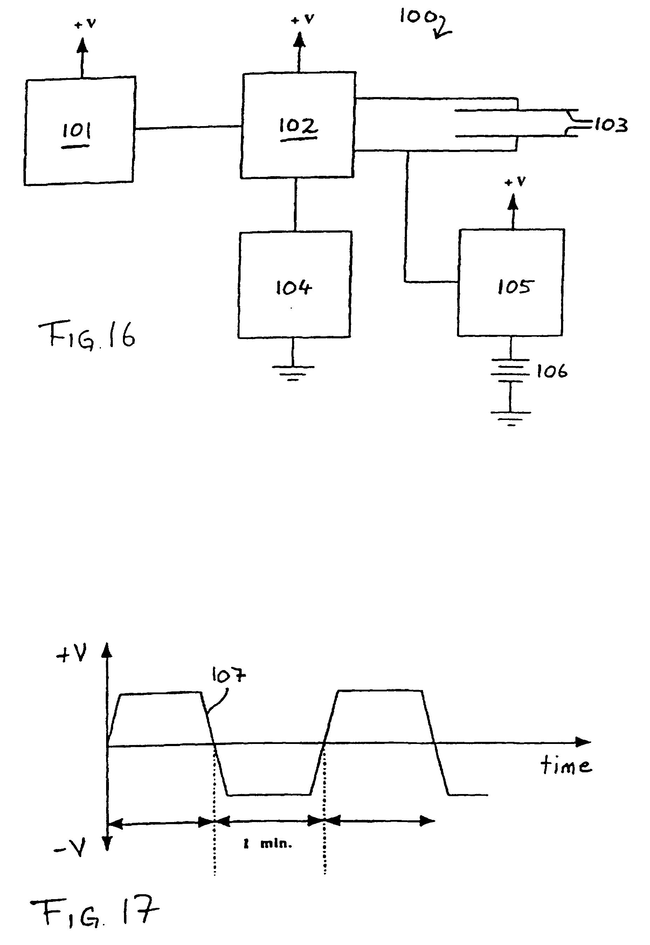

Fig. 13 shows yet a further alternative embodiment which is generally similar to those previously described but is of smaller size. Thus, the device, indicated generally at 80, has a sheet 81 having first and second halves 82,83 the undersides of which are conductive and are separated by an insulating barrier 84. A circular layer of insulating adhesive 85 holds parallel tabs 86 in a folded position and is initially covered by a release liner 87. Before use, release liner 87 is removed and first part 88 of device 80 is adhered to the adhesive 85, with a pair of parallel contacts 89 (Fig. 14) thereby making electrical contact with the folded tabs 86. The device is shown in use in Fig. 15.

Fig. 13 shows a variation on previously described devices, however, in that the underside of second half 83 of sheet 81 is provided with a layer of conductive gel 90 protected before use by a release liner 91. Before use, only the first half 82 of sheet 81 is covered by the substance to be delivered, and thus gel 90 is positioned in use in direct contact with the skin. The electronic controlling circuitry of first part 88 is adapted to deliver a substance by a direct current rather than by an alternating current. This is advantageous in certain cases, since delivery times can be shortened compared to using an alternating current. Thus, first half 82 is a permanent working electrode and second half 83 is a permanent counter electrode.

The device of Fig. 13 was tested using the local anaesthetic preparation marketed by Astra AB of Sweden as "Emla" ("Emla" is a Trade Mark for a mixture of lidocaine (lignocaine) and prilocaine). It can be used as a surface anaesthetic to produce local anaesthesia before procedures such as lumbar punctures, split skin grafting and laser treatment. It is recommended that it be applied under an occlusive dressing for at least 60 minutes before the procedure is carried out, in order for a sufficient degree of anaesthesia to be reached. When "Emla" was delivered using the device of Fig. 13, however, it was found that the same degree of anaesthesia was reached after only 15 minutes as when absorbed passively. This suggests that by enabling iontophoretic devices to be produced in lightweight inexpensive form, the devices described herein makes more attractive the use of iontophoresis in anaesthesia, which in turn allows medical procedures such as those listed above to be shortened and to become less traumatic.

The choice between direct current and alternating current depends on factors such as the nature of the substance to be delivered, and the required delivery rate.

Fig. 16 shows a block diagram of a circuit, indicated generally at 100, which is designed for the delivery of a substance using an alternating current. In circuit 100, an oscillator 101 generates signals at equal 60 second periods. These signals are fed to a H bridge 102 which provides a substantially square AC waveform voltage across the electrodes 103. Electrodes 103 in use are placed on the skin of a subject, as described above.

A current stabiliser 104 connected to the H Bridge 102 ensures that the magnitude of current is within predetermined limits, and in particular that the current does not rise above a level which could cause discomfort or skin burns.

An automatic switch 105 connected to a battery 106 which drives the circuit 100 triggers the oscillator 101 and H Bridge 102 by monitoring the flow of current from the battery 106. Before the device is applied to a subject, the circuit 100 is open and no current can flow between the electrodes 103. When the circuit is closed by application of the electrodes 103 to a subject, a small leakage current flows from the battery 106 and this triggers switch 105 to actuate the circuit 100.

Fig. 17 shows the applied voltage waveform 107 generated by the oscillator 101 and H Bridge 102. Thus, it can be seen that the waveform 107 is substantially square, having a frequency of approximately 8.3 x 10⁻³ Hz or 0.5 cycles/min (i.e. each half cycle lasts 60 seconds before the oscillator triggers the H Bridge to reverse the direction of current flow).

It will be noted, however, that the switch in voltage is not abrupt but has a saw-tooth element during the switch in polarity, and thus there is a finite time taken to change the direction of current flow between the electrodes. This has been found to allow the device to be worn more comfortably by eliminating the "tingling" sensation felt by users as the current direction changes between half-cycles.

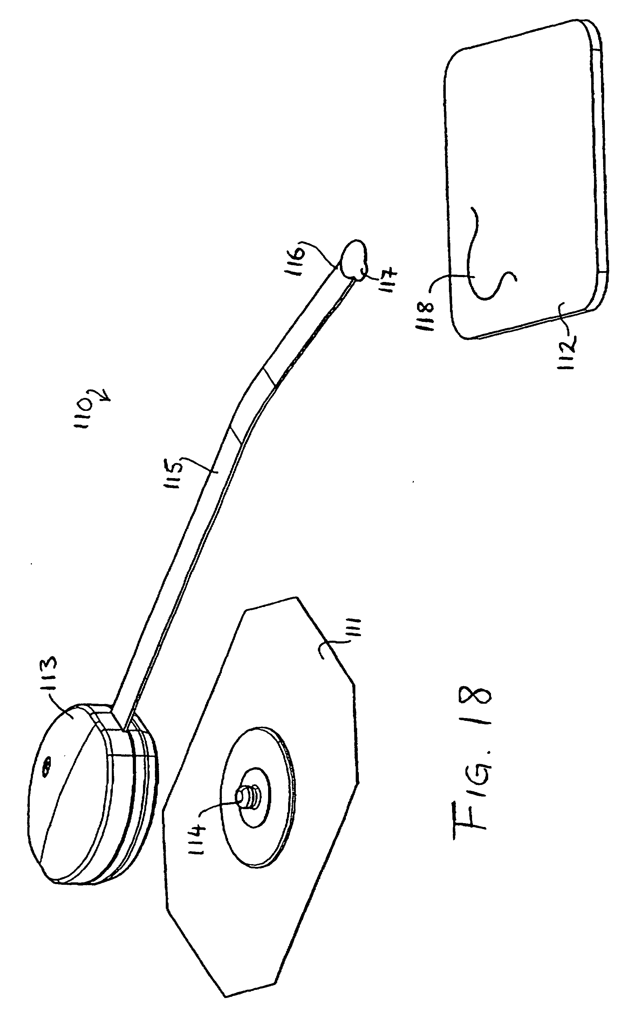

Fig. 18 shows a further embodiment of a device, indicated generally at 110, having an active electrode 111, a counter electrode 112 and an electronic control unit 113. Electronic control unit 113 is mountable on active electrode 111 by means of a snap-fit connection on the underside (not shown) of electronic control unit 113 which mates with a stud 114 mounted on active electrode 111. As well as mechanically connecting active electrode 111 and electronic control unit 113, the stud enables an electrical connection from the electronic control unit 113 to the active electrode 111.

A flexible conductive strip 115 extends from the electronic control unit 113. At its free end 116, strip 115 is provided with a release liner 117 which covers a piece of electrically conductive contact adhesive. Counter electrode 112 has a tab 118 to which the end 116 can be adhered in use to provide an electrical connection between the electronic control unit 113 and the counter electrode 112 in use.

Active electrode 111 is provided on its underside (not shown) with an absorbent sponge material which receives a solution of the substance for delivery immediately prior to use. Counter electrode 112 is a gel electrode.

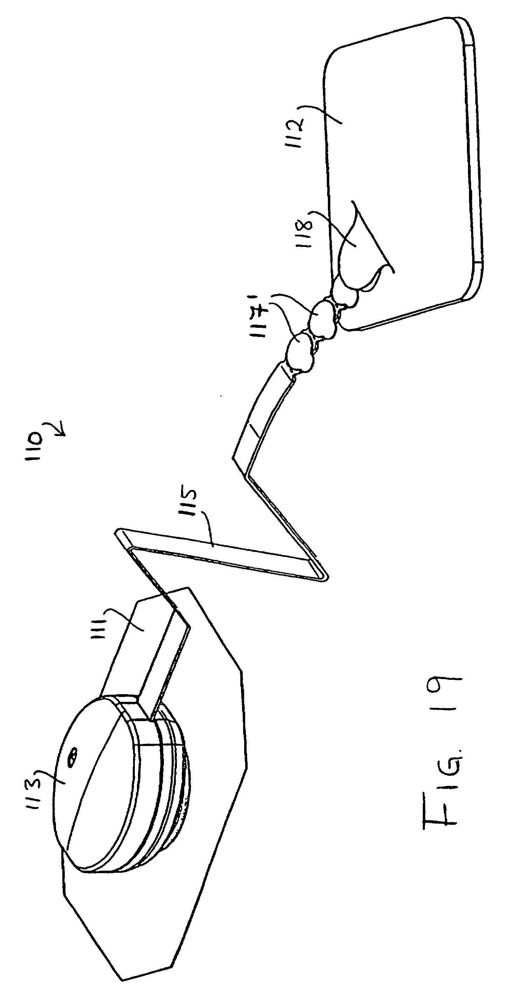

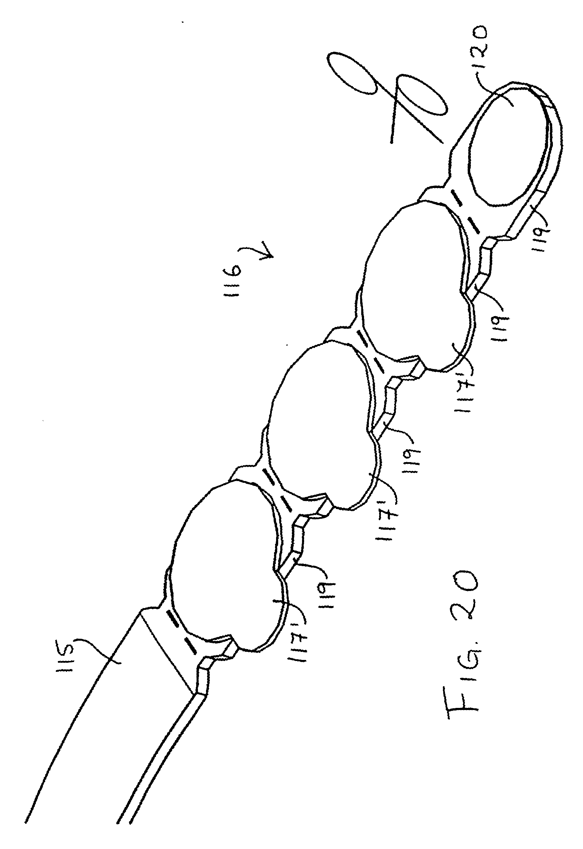

Fig. 19 shows a variation on the Fig. 18 embodiment in which like parts are represented by like reference numerals, but in which release liner 117 is replaced by a plurality of release liners 117'. Referring additionally to Fig. 20, the end 116 of strip 115 (Fig. 19) is shown in greater detail. It can be seen that the individual release liners 117' are provided on a series of perforated tear-off (or cut-off) portions 119, each release liner 117' covering a separate spot of electrically conductive contact adhesive 120.

The advantage of this embodiment is that the electronic control unit 113 can be reused a number of times (depending on the life of the battery included inside it), and connected in successive uses to fresh electrodes 111,112. After each use the electronic control unit 113 is snapped away from the active electrode 111 and the end 116 of strip 115 is disengaged from the counter electrode 112 by tearing off the endmost tear-off portion 119. The next time the device is used, the next (and now endmost) release liner 116' is removed and the underlying adhesive pressed against the tab.

Of course there is no reason why the nature of the two electrodes cannot be reversed, with the electronic control unit 113 mounted on a counter electrode and connected by adhesive means to an active electrode.

As a variation on the device of Fig. 18, the strip may be separate from the control unit, and it may be provided at both ends with an adhesive pad. One end of the adhesive pad may then be connected adhesively to an electrical contact point on the control unit and the other end may be connected adhesively to the counter electrode as described in relation to Figs. 18-20.

Alternatively, the counter electrode could be supplied with a connecting strip extending therefrom for adhesive connection to a control unit (as opposed to the Fig. 18 embodiment wherein the strip 115 extends from the control unit 113 for adhesive connection to the electrode 112).

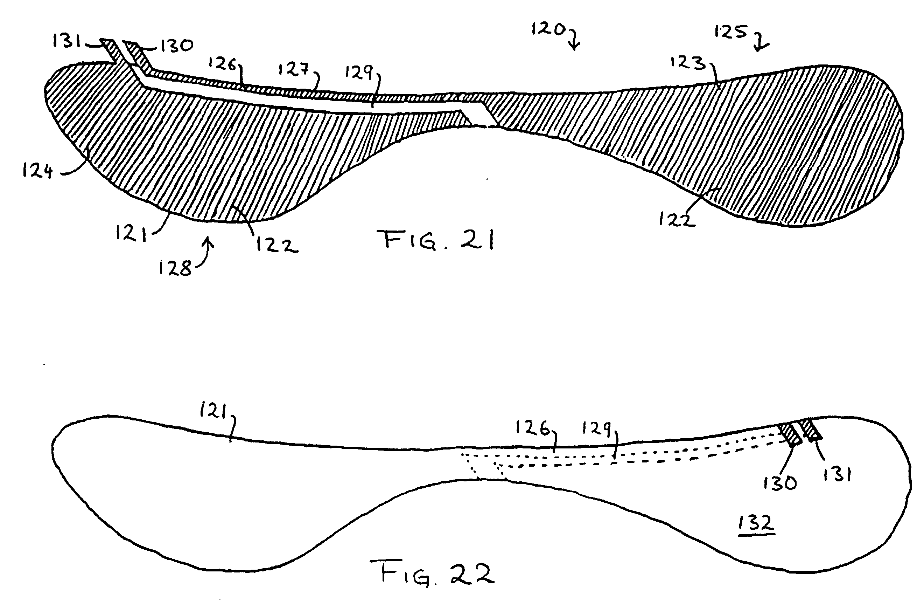

Fig. 21 shows an embodiment of an improved electrode assembly according to the invention for use in a device for the delivery of a substance to a subject, similar in many respects to the embodiment shown in Figs. 8-10. In Fig. 21 there is indicated, generally at 120, the underside of a tissue paper sheet 121 printed with conductive ink 122 to form two spaced-apart electrodes 123,124.

Electrode 123 substantially covers one half 125 of sheet 121 but extends in a continuous strip 126 along an edge 127 of the other half 128 of the sheet 121. Electrode 124 substantially covers this other half 128 but stops short of the edge 127 so that a gap 129 is present between the electrodes 123, 124. At the end of the strip 126, electrode 123 terminates in a tab 130. Electrode 124 is provided with an adjacent tab 131.

Referring next to Fig. 22, the top side of tissue paper 121 can be seen, with strip 126 and gap 129 indicated by dotted lines. The tabs 130,131 are folded over and adhered onto the top surface 132 of sheet 121, providing electrically conductive contacts against which the electronic control unit of Fig. 7 can be adhesively mounted as previously described. The embodiment of Figs. 20 and 21 is advantageous in that it obviates the necessity of providing a connecting strip (such as the strip 62 shown in Fig. 8), since the printed areas of the electrodes 123,124 are shaped to make such a strip redundant.

## Claims

1. An improved electrode assembly for use in a device for the delivery of a substance to a subject, comprising:
a) a flexible substrate (121) having a first surface (120) and a second opposed surface (132);
b) a pair of spaced apart electrodes (123, 124) applied to the first surface (120) of the substrate (121);
c) a tab (130, 131) extending from each electrode (123, 124) comprised of electrically conductive material; and
d) a power source located within a housing having a pair of spaced apart electrical contacts located on the bottom surface of the housing;
whereby the tabs (130, 131) are folded over onto the opposed surface (132) of the substrate (121) and the power source is fixed to the opposed surface (132) so that each electrical contact is in electrical communication with one of the tabs (130, 131).

2. An assembly according to Claim 1, wherein the substrate comprises a non-woven tissue paper (121).

3. An assembly according to Claim 1 or 2, wherein the electrodes (123, 124) are comprised of electrically conductive ink (122).

4. An assembly according to any one of Claims 1-3, wherein the electrodes (123, 124) are printed onto the substrate (121).

5. An assembly according to any one of Claims 1-4, wherein the power source is fixed to the opposed surface (132) of the substrate (121) by means of an electrically conductive adhesive.

6. An assembly according to any one of Claims 1-5, wherein the assembly further comprises means for retaining the substrate to the skin of a mammal.

7. An assembly according to Claim 6, wherein the retaining means is an adhesive.

8. An assembly according to any one of Claims 1-7, wherein the power source further comprises an electrical circuit capable of generating an alternating voltage.

9. An assembly according to Claim 8, wherein the voltage alternates between the electrodes (123, 124).

10. An assembly according to Claim 8 or 9, wherein the voltage alternates with a period of about 20 seconds to about 10 minutes.

11. An assembly according to Claim 10, wherein the voltage alternates with a period of about 1 minute to about 5 minutes.

12. An assembly according to any one of Claims 8-11, wherein the alternating voltage defines a substantially square waveform having a sawtooth component during the transition between polarities.

## Patentansprüche

1. Verbesserte Elektrodenanordnung zur Verwendung in einer Vorrichtung zur Abgabe einer Substanz an ein Subjekt umfassend:
a) ein nachgiebiges Substrat (121), welches eine erste Oberfläche (120) und eine zweite gegenüberliegende Oberfläche (132) aufweist;
b) ein Paar von voneinander beabstandeten Elektroden (123, 124), angebracht an der ersten Oberfläche (120) des Substrates (121);
c) einen Lappen (130, 131), der sich von jeder Elektrode (123, 124) erstreckt, welcher aus elektrisch leitfähigem Material besteht; und
d) eine Energiequelle, welche sich in einem Gehäuse befindet, und ein Paar voneinander beabstandeten elektrischen Kontakten aufweist, welche sich an der Bodenoberfläche des Gehäuses befinden;
wobei die Lappen (130, 131) über die gegenüberliegende Oberfläche (132) des Substrates (121) gefaltet sind und die Energiequelle an der gegenüberliegenden Oberfläche (132) befestigt ist, so dass sich jeder elektrische Kontakt in Verbindung mit einem der Lappen (130, 131) befindet.

2. Anordnung gemäß Anspruch 1, wobei das Substrat ein nicht-gewobenes Gewebepapier (121) aufweist.

3. Anordnung gemäß einem der Ansprüche 1 oder 2, wobei die Elektroden (123, 124) aus elektrisch leitfähiger Tinte (122) aufgebaut sind.

4. Anordnung gemäß einem der Ansprüche 1 bis 3, wobei die Elektroden (123, 124) auf das Substrat (121) gedruckt werden.

5. Anordnung gemäß einem der Ansprüche 1 bis 4, wobei die Energiequelle auf der gegenüberliegenden Oberfläche (132) des Substrats (121) mittels eines elektrisch leitfähigen Klebers befestigt ist.

6. Anordnung gemäß einem der Ansprüche 1 bis 5, wobei die Anordnung weiter Mittel zur Rückhaltung des Substrats auf der Haut eines Säugetieres aufweist.

7. Anordnung gemäß Anspruch 6, wobei die Rückhaltemittel einen Kleber darstellen.

8. Anordnung gemäß einem der Ansprüche 1 bis 7, wobei die Energiequelle weiter einen elektrischen Schaltkreis aufweist, der dazu in der Lage ist, einen Wechselstrom zu erzeugen.

9. Anordnung gemäß Anspruch 8, wobei die Spannung zwischen den Elektroden (123, 124) wechselt.

10. Anordnung gemäß Anspruch 8 oder 9, wobei die Spannung mit einer Periode von ungefähr 20 Sekunden bis 10 Minuten wechselt.

11. Anordnung gemäß Anspruch 10, wobei die Spannung mit einer Periode von ungefähr 1 Minute bis 5 Minuten wechselt.

12. Anordnung gemäß einem der Ansprüche 8 bis 11, wobei die Wechselspannung eine im Wesentlichen rechteckige Wellenform vorgibt, welche eine Sägezahnkomponente während dem Übergang zwischen der Polaritäten aufweist.

## Revendications

1. Ensemble amélioré d'électrodes à utiliser dans un dispositif destiné à la délivrance d'une substance à un sujet, comprenant :
a) un substrat souple (121) présentant une première surface (120) et une seconde surface opposée (132) ;
b) une paire d'électrodes placées à distance (123, 124) appliquées à la première surface (120) du substrat (121) ;
c) une plaquette (130, 131) s'étendant à partir de chaque électrode (123, 124) constituée d'un matériau électriquement conducteur ; et
d) une source d'alimentation électrique placée à l'intérieur d'un logement comportant une paire de contacts électriques placés séparément sur la surface inférieure du logement ;
de façon que les plaquettes (130, 131) soient repliées sur la surface opposée (132) du substrat (121) et que la source d'alimentation électrique soit fixée sur la surface opposée (132) de façon que chaque contact électrique se trouve en communication électrique avec l'une des plaquettes (130, 131).

2. Ensemble selon la revendication 1, dans lequel le substrat comprend un papier-tissu non tissé (121).

3. Ensemble selon la revendication 1 ou 2, dans lequel les électrodes (123, 124) sont composées d'une encre électriquement conductrice (122).

4. Ensemble selon l'une quelconque des revendications 1 à 3, dans lequel les électrodes (123, 124) sont imprimées sur le substrat (121).

5. Ensemble selon l'une quelconque des revendications 1 à 4 dans lequel la source d'alimentation électrique est fixée sur la surface opposée (132) du substrat (121) au moyen d'un adhésif électriquement conducteur.

6. Ensemble selon l'une quelconque des revendications 1 à 5, dans lequel l'ensemble comporte, de plus, des moyens pour retenir le substrat sur la peau d'un mammifère.

7. Ensemble selon la revendication 6, dans lequel le moyen de retenue est un adhésif.

8. Ensemble selon l'une quelconque des revendications 1 à 7 dans lequel la source d'alimentation électrique comprend, de plus, un circuit électrique capable de générer une tension alternative.

9. Ensemble selon la revendication 8 , dans lequel la tension alterne entre les électrodes (123, 124).

10. Ensemble selon la revendication 8 ou 9, dans lequel la tension alterne avec une période de 20 secondes environ à 10 minutes environ.

11. Ensemble selon la revendication 10 dans lequel la tension alterne avec une période de 1 minute environ à 5 minutes environs.

12. Ensemble selon l'une quelconque des revendications 8 à 11 dans lequel la tension alternative définit une forme d'onde essentiellement carrée présentant une composante en dent de scie pendant la transition entre les polarités.
